# EUROPEAN PATENT APPLICATION

(11) **EP 4 289 877 A1**
(43) Date of publication of application: **13.12.2023**
(21) Application number: 22749838.3
(22) Date of filing: 07.02.2022
(51) Int. Cl.: C08F 290/06, C08F 220/10, C08F 220/26, A61K 47/58

(54) **NOVEL COPOLYMER**

(30) Priority: 05.02.2021 JP 2021017807
(71) Applicant: KOWA COMPANY, LTD., Naka-ku Nagoya-shi Aichi 460-8625 (JP)
(72) Inventor: KANAYA, Ryo, Higashimurayama-shi, Tokyo 189-0022 (JP); SUZUKI, Kenichi, Higashimurayama-shi, Tokyo 189-0022 (JP); CHIDA, Tsukasa, Higashimurayama-shi, Tokyo 189-0022 (JP); FUJIMAKI, Nobuhiro, Higashimurayama-shi, Tokyo 189-0022 (JP); ECHIGO, Marina, Higashimurayama-shi, Tokyo 189-0022 (JP); MIURA, Seiji, Higashimurayama-shi, Tokyo 189-0022 (JP)
(74) Representative: Wächtershäuser & Hartz Patentanwaltspartnerschaft mbB
(86) International application number: PCT/JP2022/004592
(87) International publication number: WO 2022/168967

(57) **Abstract**

The present invention provides a novel copolymer available for a drug delivery technique. More particularly, a novel copolymer for a drug delivery carrier targeting a tumor is provided.

The present invention relates to a copolymer comprising structural units represented by the following formulas (A), (B), and (C): 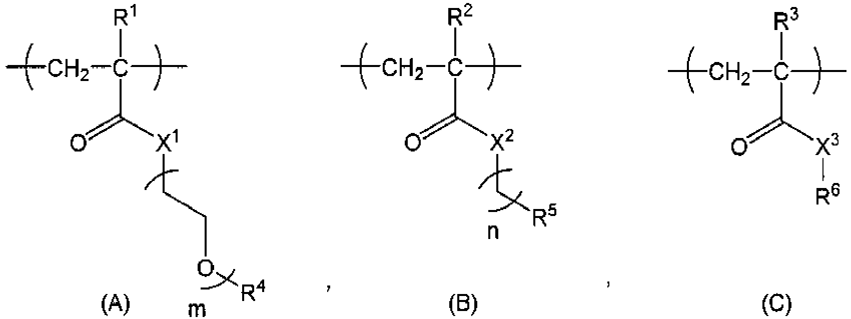 wherein R¹, R², and R³ are the same or different and represent a hydrogen atom or a C₁₋₃ alkyl group, R⁴ represents a C₁₋₃ alkyl group, R⁵ represents a hydrogen atom, a C₁₋₁₈ alkyl group, a 3- to 8-membered cycloalkyl group optionally having a substituent, an adamantyl group, a C₆₋₁₈ aryl group optionally having a substituent, or a 5- to 10-membered heteroaryl group optionally having a substituent, X¹, X², and X³ are the same or different and represent an oxygen atom, a sulfur atom, or N-R⁷, R⁶ represents a hydrogen atom, a leaving group, or a linker, R⁷ represents a hydrogen atom or a C₁₋₃ alkyl group, m represents an integer of 1 to 100, and n represents an integer of 0 to 3.

## Description

### Technical Field

The present invention relates to a novel copolymer available for a drug delivery technique. More particularly, the present invention relates to a copolymer for a drug delivery carrier targeting a tumor, a pharmaceutical composition in which a physiologically active substance such as an anticancer agent is supported on the copolymer, and a medicine containing the composition.

### Background Art

In recent years, research on drug delivery system (DDS) has been energetically conducted as a technique for efficiently and safely delivering a drug to a disease site. Among them, there is an increasing demand for DDS employing nanoparticles as drug delivery carriers as a technique for enhancing selectivity of drug accumulation by utilizing structural properties of a disease site.

For example, in a solid cancer tissue, since a structure of a neovascular vessel (tumor blood vessel) is immature as compared with a normal blood vessel, a cell gap of about several hundred nm is generated in the vascular endothelium, which allows permeation of a large amount of substances. Due to this structural feature, it is known that a polymeric compound containing nanoparticles selectively permeates a tumor blood vessel and accumulates in a solid cancer tissue. Furthermore, in a solid cancer tissue, a lymphatic system involved in excretion of polymers malfunctions, so that permeated nanoparticles are continuously retained in the tissue (Enhanced permeability and retention effect, EPR effect). Since a general low molecular drug leaks out of a blood vessel due to membrane permeation of vascular cells, it is non-selectively distributed in a tissue and does not accumulate in a solid cancer tissue. According to a methodology of the EPR effect, nanoparticle-based drug delivery results in improved tissue selectivity to a solid cancer in distribution of a drug, since distribution to a tissue is governed by permeability of a vascular endothelial cell gap. Therefore, the EPR effect is a promising academic support in development of nanotechnology-applied medicines (nanomedicines) targeting a solid cancer.

It is believed that a drug delivery process in the EPR effect is via blood flow and an extravasation process of nanoparticles is passive. Therefore, in order to maximize accumulation of nanoparticles against a solid cancer, it is important to impart a molecular design that can withstand long-term blood retention to constituent components of nanoparticles to be drug delivery carriers. Drug delivery carriers are therefore required to have an ability to avoid barriers such as non-specific interactions with blood constituent components, foreign body recognition by the reticuloendothelial system (RES) in the liver, spleen, and lung, and glomerular filtration in the kidney. In addition, it is known that these barriers can be overcome by optimization of particle properties such as particle size and surface modification with a biocompatible polymer. For example, it is desirable that the particle size of the drug delivery carrier be greater than about 6 nm, which is a threshold for renal clearance, and less than 200 nm, which may avoid recognition by the RES.

The particle size of the drug delivery carrier is also known to affect tissue permeation at a disease site. For example, anticancer activity of drug-encapsulating nanoparticles having particle sizes of 30 nm, 50 nm, 70 nm, and 100 nm, which exhibit equivalent blood retention, has been comparatively studied, and it has been revealed that drug-encapsulating nanoparticles having a particle size of 30 nm reach a deep part of a disease site, and thus exhibit the highest therapeutic effect (Non Patent Literature 1). Therefore, it would be desirable that a particle size of nanoparticles for a drug delivery carrier targeting a solid cancer be as small as possible to the extent that renal clearance can be avoided.

As the nanoparticles for a drug delivery carrier, methods using colloidal dispersions such as liposomes, emulsions, or nanoparticles, methods using biologically derived raw materials such as albumin, methods using natural polymers such as natural polysaccharides, or methods using synthetic polymers, have been developed. Among them, a synthetic polymer is widely used as a constituent of a drug delivery carrier because it is possible to prepare nanoparticles whose particle size is precisely controlled by appropriately selecting a monomer as a constituent and a synthesis method.

For example, a method for utilizing an amphiphilic block copolymer composed of a hydrophilic segment and a hydrophobic segment as a drug delivery carrier is disclosed. The block copolymer spontaneously associates in an aqueous medium by, for example, a hydrophobic interaction between molecules as a driving force to form core-shell type nanoparticles (polymeric micelles). It is known that it is possible to encapsulate a low molecular drug in or bind it to the hydrophobic segment of the polymeric micelle, and the obtained drug-encapsulating polymeric micelle exhibits high blood stability, and exhibits high anticancer activity as compared with administration of a solution of a low molecular drug due to selective accumulation in a solid cancer through the EPR effect (Patent Literature 1). However, since the polymeric micelle is an associate of multiple molecules, a particle size of about 30 nm is a lower limit value that can be prepared, and it is difficult to finely control a size in the vicinity of a particle size of 10 nm that can avoid the influence of renal clearance.

Meanwhile, among nanoparticles formed of a synthetic polymer, those which form particles by, for example, chemical crosslinking, hydrophobic interaction, or ionic bonding in a single chain as a driving force (hereinafter abbreviated as single chain nanoparticles (SCNPs)) are known to form nanoparticles having a small particle size of 20 nm or less (Non Patent Literature 2). Therefore, although the SCNP is expected to be useful as a drug delivery carrier, a technique for precisely controlling its particle size has not been found so far.

### Citation List

### Patent Literature

Patent Literature 1: JP 3270592 B2

### Non Patent Literature

Non Patent Literature 1: H. Cabral et al., Nat. Nanotechnol. 6 815-823(2011)
Non Patent Literature 2: Jose A. Pomposo, Single-Chain Polymer Nanoparticles:Synthesis, Characterization, Simulations, and Applications(2017)

### Summary of the Invention

### Technical Problem

It is an object of the present invention to provide a copolymer for a drug delivery carrier targeting a tumor. More particularly, it is an object of the present invention to provide a copolymer for a drug delivery carrier available for improvement in blood retention and/or tumor accumulation of a drug.

### Solution to Problem

In order to achieve the above-mentioned objects, the present inventors energetically studied, and found a property that a terpolymer of an acrylic acid derivative forms an SCNP in water. In addition, the present inventors succeeded in creating a novel polymer for a drug delivery carrier, which is capable of precisely controlling a particle size of an SCNP at a minute scale of 20 nm or less and about 10 nm, and has high tumor accumulation. Furthermore, when an anticancer agent was supported on the polymer and the polymer was administered to a model mouse for colon cancer subcutaneous transplantation, an excellent antitumor effect was confirmed.

The present invention relates to the following:
[1] A copolymer comprising structural units represented by the following formulas (A), (B), and (C): 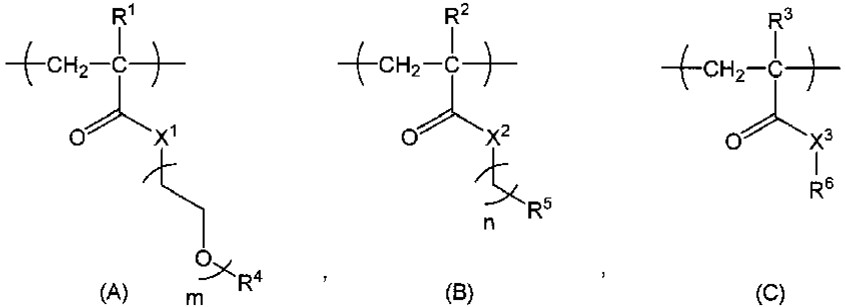 wherein R¹, R², and R³ are the same or different and represent a hydrogen atom or a C₁₋₃ alkyl group, R⁴ represents a C₁₋₃ alkyl group, R⁵ represents a hydrogen atom, a C₁₋₁₈ alkyl group, a 3- to 8-membered cycloalkyl group optionally having a substituent, an adamantyl group, a C₆₋₁₈ aryl group optionally having a substituent, or a 5- to 10-membered heteroaryl group optionally having a substituent, X¹, X², and X³ are the same or different and represent an oxygen atom, a sulfur atom, or N-R⁷, R⁶ represents a hydrogen atom, a leaving group, or a linker, R⁷ represents a hydrogen atom or a C₁₋₃ alkyl group, m represents an integer of 1 to 100, and n represents an integer of 0 to 3.
[2] A copolymer formed by polymerization of three monomers represented by the following general formulas (1) to (3): 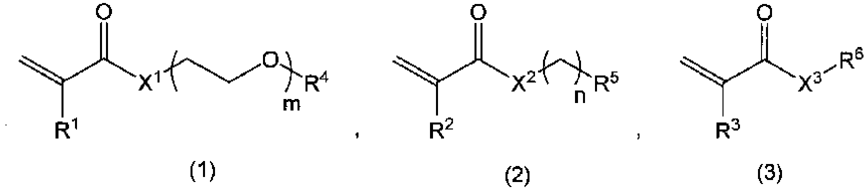 wherein R¹, R², and R³ are the same or different and represent a hydrogen atom or a C₁₋₃ alkyl group, R⁴ represents a C₁₋₃ alkyl group, R⁵ represents a hydrogen atom, a C₁₋₁₈ alkyl group, a 3- to 8-membered cycloalkyl group optionally having a substituent, an adamantyl group, a C₆₋₁₈ aryl group optionally having a substituent, or a 5- to 10-membered heteroaryl group optionally having a substituent, X¹, X², and X³ are the same or different and represent an oxygen atom, a sulfur atom, or N-R⁷, R⁶ represents a hydrogen atom, a leaving group, or a linker, R⁷ represents a hydrogen atom or a C₁₋₃ alkyl group, m represents an integer of 1 to 100, and n represents an integer of 0 to 3.
[3] The copolymer according to [1] or [2], wherein R¹ is a hydrogen atom.
[4] The copolymer according to any one of [1] to [3], wherein R² is a hydrogen atom.
[5] The copolymer according to any one of [1] to [4], wherein R³ is a hydrogen atom.
[6] The copolymer according to any one of [1] to [5], wherein R⁴ is a methyl group.
[7] The copolymer according to any one of [1] to [6], wherein R⁵ is a C₆₋₁₈ aryl group optionally having a substituent.
[8] The copolymer according to any one of [1] to [7], wherein R⁵ is a phenyl group.
[9] The copolymer according to any one of [1] to [8], wherein R⁶ is a hydrogen atom.
[10] The copolymer according to any one of [1] to [8], wherein the leaving group of R⁶ is the following formula (4) : 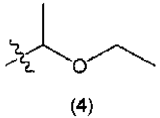
[11] The copolymer according to any one of [1] to [8], wherein the linker of R⁶ is selected from the following formulas (5) to (7): 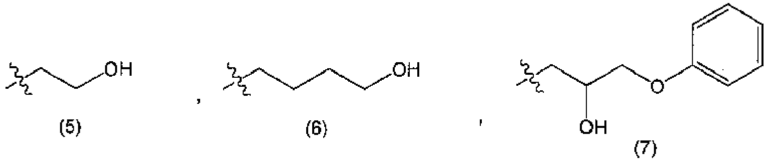
[12] The copolymer according to any one of [1] to [11], wherein X¹ is an oxygen atom.
[13] The copolymer according to any one of [1] to [12], wherein X² is an oxygen atom.
[14] The copolymer according to any one of [1] to [13], wherein X³ is an oxygen atom.
[15] The copolymer according to any one of [1] to [14], wherein m is an integer of 4 to 22.
[16] The copolymer according to any one of [1] to [15], wherein n is 1.
[17] The copolymer according to any one of [1] to [16], wherein a ratio of structural units (A), (B), and (C) is composed of 0.01 to 100 parts by mass of (B) and 0.1 to 100 parts by mass of (C) with respect to 1 part by mass of (A).
[18] The copolymer according to any one of [2] to [16], wherein 0.01 to 100 parts by mass of monomer (2) and 0.1 to 100 parts by mass of monomer (3) are polymerized with respect to 1 part by mass of monomer (1).
[19] The copolymer according to any one of [1] to [18], wherein a number average molecular weight is 5 000 to 150 000.
[20] A single chain nanoparticle including the copolymer according to any one of [1] to [19].
[21] A pharmaceutical composition including the copolymer according to any one of [1] to [19].

### Advantageous Effects of Invention

As will be evident from Examples described below, an SCNP obtained by self-association of the copolymer of the present invention supporting an anticancer agent exhibited a tumor growth suppressing effect in a mouse tumor-bearing model, and therefore can be applied as a therapeutic agent for a malignant tumor. Since the SCNP obtained by self-association of the copolymer of the present invention supporting an anticancer agent has a high tumor growth suppressing effect at a low dose, it is possible to provide a therapeutic agent for a malignant tumor capable of achieving both enhancement of a pharmacological action and suppression of side effects.

### Brief Description of Drawings

Fig. 1 is a diagram showing a ¹H-NMR spectrum of a copolymer obtained in Example 1 measured by using nuclear magnetic resonance (NMR).
Fig. 2 is a diagram showing a chromatogram of a copolymer obtained in Example 1 obtained by gel permeation chromatography (GPC).
Fig. 3 is a diagram showing a particle size measurement result (scattering intensity distribution) in dynamic light scattering (DLS) for a copolymer before encapsulating DACHPt (Example 69) and a DACHPt-encapsulating SCNP (Example 70).
Fig. 4 is a diagram showing a change in relative tumor volume when an oxaliplatin solution or a DACHPt-encapsulating SCNP (Example 70) was administered three times every other day to a mouse model obtained by subcutaneously transplanting a mouse colon cancer cell line (C26) into the back.

### Description of Embodiments

Terms used herein are used in the meanings commonly used in the art unless otherwise specified. Hereinafter, the present invention will be described in more detail.

In the present description, the term "nanoparticle" refers to a structure exhibiting a particle size of 100 nm or less.

In the present description, the term "single chain nanoparticle (SCNP)" refers to a nanoparticle formed by, for example, chemical crosslinking, hydrophobic interaction, or ionic bonding in a single chain as a driving force. The SCNP often has a relatively small particle size of 20 nm or less among those of nanoparticles.

In the present description, the term "initiator" means an initiator of thermal radical polymerization such as an azo compound or a peroxide.

In the present description, the term "chain transfer agent" refers to a compound that causes a chain transfer reaction in radical polymerization, and is preferably a compound having a thiocarbonyl group.

In the present description, the term "C₁₋₃ alkyl group" means a linear or branched alkyl group having 1 to 3 carbon atoms, and examples thereof include a methyl group, an ethyl group, an n-propyl group, and an isopropyl group.

In the present description, the term "C₁₋₁₈ alkyl group" means a linear or branched alkyl group having 1 to 18 carbon atoms, and examples thereof include a methyl group, an ethyl group, an n-propyl group, an isopropyl group, an n-butyl group, an isobutyl group, a sec-butyl group, a tert-butyl group, a pentyl group, a hexyl group, a heptyl group, an octyl group, a nonyl group, a decyl group, an undecyl group, a dodecyl group, a tridecyl group, a tetradecyl group, a pentadecyl group, a hexadecyl group, a heptadecyl group, and an octadecyl group.

In the present description, the term "3- to 8-membered cycloalkyl group optionally having a substituent" means a cyclic alkyl group having 3 to 8 carbon atoms, and examples thereof include a cyclopropyl group, a cyclobutyl group, a cyclopentyl group, a cyclohexyl group, a cycloheptyl group, and a cyclooctyl group. The substituent is not particularly limited, and examples thereof include a halogen atom, an alkyl group having 1 to 6 carbon atoms, an alkenyl group having 2 to 6 carbon atoms, an alkynyl group having 2 to 6 carbon atoms, a hydroxyl group, an alkoxy group having 1 to 6 carbon atoms, an amino group, an alkylamino group having 1 to 6 carbon atoms, a di-alkylamino group having 1 to 6 carbon atoms in which alkyl groups are the same or different, a thiol group, an alkylthio group having 1 to 6 carbon atoms, a carboxyl group, an alkoxycarbonyl group having 1 to 6 carbon atoms, and a carbamoyl group.

In the present description, the term "C₆₋₁₈ aryl group optionally having a substituent" means a monocyclic or polycyclic condensed aromatic hydrocarbon group, and examples thereof include a phenyl group, a naphthyl group, an anthracenyl group, a phenanthrenyl group, a triphenylenyl group, a pyrenyl group, a chrysenyl group, and a naphthacenyl group. The term "C₆₋₁₄ aryl group optionally having a substituent" means a monocyclic or polycyclic condensed aromatic hydrocarbon group, and examples thereof include a phenyl group, a naphthyl group, an anthracenyl group, and a phenanthrenyl group. The substituent is not particularly limited, and examples thereof include a halogen atom, an alkyl group having 1 to 6 carbon atoms, an alkenyl group having 2 to 6 carbon atoms, an alkynyl group having 2 to 6 carbon atoms, a hydroxyl group, an alkoxy group having 1 to 6 carbon atoms, an amino group, an alkylamino group having 1 to 6 carbon atoms, a di-alkylamino group having 1 to 6 carbon atoms in which alkyl groups are the same or different, a thiol group, an alkylthio group having 1 to 6 carbon atoms, a carboxyl group, an alkoxycarbonyl group having 1 to 6 carbon atoms, and a carbamoyl group.

In the present description, the term "5- to 10-membered heteroaryl group optionally having a substituent" means a 5- to 10-membered monocyclic aromatic heterocyclic group or a fused aromatic heterocyclic group containing 1 to 4 heteroatoms selected from a nitrogen atom, an oxygen atom, and a sulfur atom, other than a carbon atom, as atoms constituting the ring. Examples of the monocyclic aromatic heterocyclic group include a furyl group, a thienyl group, a pyrrolyl group, a pyridinyl group, a pyrazinyl group, a pyrimidinyl group, a pyridazinyl group, an imidazolyl group, a pyrazyl group, a thiallyl group, an oxazolyl group, an isoxazolyl group, a 1,3,4-thiadiazolyl group, a 1,2,3-triazolyl group, a 1,2,4-triazolyl group, and a tetrazolyl group. Examples of the fused aromatic heterocyclic group include a benzofuranyl group, a benzothiophenyl group, a quinoxalinyl group, an indolyl group, an isoindolyl group, an isobenzofuranyl group, a chromanyl group, a benzimidazolyl group, a benzothiazolyl group, a benzoxazolyl group, a quinolyl group, and an isoquinolinyl group. The term "6- to 10-membered heteroaryl group optionally having a substituent" means a 6- to 10-membered monocyclic aromatic heterocyclic group or a fused aromatic heterocyclic group containing 1 to 4 heteroatoms selected from a nitrogen atom, an oxygen atom, and a sulfur atom, other than a carbon atom, as atoms constituting the ring. Examples of the monocyclic aromatic heterocyclic group include a pyridinyl group, a pyrazinyl group, a pyrimidinyl group, and a pyridazinyl group, for example. Examples of the fused aromatic heterocyclic group include a benzofuranyl group, a benzothiophenyl group, a quinoxalinyl group, an indolyl group, an isoindolyl group, an isobenzofuranyl group, a chromanyl group, a benzimidazolyl group, a benzothiazolyl group, a benzoxazolyl group, a quinolyl group, and an isoquinolinyl group. The substituent is not particularly limited, and examples thereof include a halogen atom, an alkyl group having 1 to 6 carbon atoms, an alkenyl group having 2 to 6 carbon atoms, an alkynyl group having 2 to 6 carbon atoms, a hydroxyl group, an alkoxy group having 1 to 6 carbon atoms, an amino group, an alkylamino group having 1 to 6 carbon atoms, a di-alkylamino group having 1 to 6 carbon atoms in which alkyl groups are the same or different, a thiol group, an alkylthio group having 1 to 6 carbon atoms, a carboxyl group, an alkoxycarbonyl group having 1 to 6 carbon atoms, and a carbamoyl group.

In the present description, examples of the "halogen atom" include a fluorine atom, a chlorine atom, a bromine atom, and an iodine atom.

In the copolymer of the present invention, the structural unit (A) functions as a unit that imparts hydrophilicity, and the structural unit (B) functions as a unit that imparts hydrophobicity. The structural unit (C) functions as a scaffold to which active ingredients (drug, physiologically active substance) and the copolymer are bound. Having these three structural units serves to imparting the copolymer of the present invention a property of forming an SCNP in water, and to facilitating the formed SCNP to precisely control particle size at a minute scale of 20 nm or less, and to function as a drug delivery carrier having high tumor accumulation.

R¹ in the structural unit (A) represents a hydrogen atom or a C₁₋₃ alkyl group, and is preferably a hydrogen atom or a methyl group, and is preferably a hydrogen atom, an ethyl group, an n-propyl group, or an isopropyl group, and more preferably a hydrogen atom.

X¹ represents an oxygen atom, a sulfur atom, or N-R⁷, and is preferably an oxygen atom, a sulfur atom, or NH, and more preferably an oxygen atom.
m represents an integer of 1 to 100, and is preferably an integer of 3 to 100, and is preferably 3 to 80, more preferably 4 to 60, still more preferably 4 to 40, and yet more preferably 4 to 22 from the viewpoint of imparting good hydrophilicity.

R⁴ represents a C₁₋₃ alkyl group, specifically a methyl group, an ethyl group, an n-propyl group, or an isopropyl group, preferably a methyl group or an ethyl group, and more preferably a methyl group.

R² in the structural unit (B) represents a hydrogen atom or a C₁₋₃ alkyl group, and is preferably a hydrogen atom or a methyl group, and is preferably a hydrogen atom, an ethyl group, an n-propyl group, or an isopropyl group, and more preferably a hydrogen atom.

X² represents an oxygen atom, a sulfur atom, or N-R⁷, and is preferably an oxygen atom, a sulfur atom, or NH, and more preferably an oxygen atom.
n represents an integer of 0 to 3, preferably an integer of 1 to 3, and more preferably 1.

R⁵ represents a hydrogen atom, a C₁₋₁₈ alkyl group, a 3-to 8-membered cycloalkyl group optionally having a substituent, an adamantyl group, a C₆₋₁₈ aryl group optionally having a substituent, or a 5- to 10-membered heteroaryl group optionally having a substituent, and from the viewpoint of imparting hydrophobicity to the structural unit (B), a C₁₋₁₈ alkyl group, a 3- to 8-membered cycloalkyl group optionally having a substituent, an adamantyl group, a C₆₋₁₈ aryl group optionally having a substituent, or a 5-to 10-membered heteroaryl group optionally having a substituent is preferable, a C₁₋₁₈ alkyl group, a 3- to 8-membered cycloalkyl group optionally having a substituent, an adamantyl group, a C₆₋₁₈ aryl group optionally having a substituent, or a 5- to 10-membered heteroaryl group optionally having a substituent is more preferable, and a C₁₋₁₈ alkyl group, a 3- to 8-membered cycloalkyl group, an adamantyl group, or a C₆₋₁₈ aryl group is still more preferable. In addition, a 3- to 8-membered cycloalkyl group optionally having a substituent, an adamantyl group, a C₆₋₁₄ aryl group optionally having a substituent, or a 6-to 10-membered heteroaryl group optionally having a substituent is also preferable. Here, the substituent is preferably one or two or more selected from a halogen atom, an alkyl group having 1 to 6 carbon atoms, an alkenyl group having 2 to 6 carbon atoms, and an alkynyl group having 2 to 6 carbon atoms.

R³ in the structural unit (C) represents a hydrogen atom or a C₁₋₃ alkyl group, and is preferably a hydrogen atom or a methyl group, and is preferably a hydrogen atom, an ethyl group, an n-propyl group, or a 1-propyl group, and more preferably a hydrogen atom.

X³ represents an oxygen atom, a sulfur atom, or N-R⁷, and is preferably an oxygen atom, a sulfur atom, or NH, and more preferably an oxygen atom.

R⁶ represents a hydrogen atom, a leaving group, or a linker. This leaving group is a group that can detach when the structural unit (C) binds to a drug (physiologically active substance), and the linker is a group that can be used for crosslinking when the structural unit (C) binds to a drug (physiologically active substance). As the leaving group or linker, a C₁₋₁₈ alkyl group optionally having a substituent, a 3- to 8-membered cycloalkyl group optionally having a substituent, or a C₇₋₁₉ aralkyl group optionally having a substituent is preferable. Here, examples of the substituent include a halogen atom, an alkyl group having 1 to 6 carbon atoms, an alkenyl group having 2 to 6 carbon atoms, an alkynyl group having 2 to 6 carbon atoms, a hydroxyl group, an alkoxy group having 1 to 6 carbon atoms, an amino group, an alkylamino group having 1 to 6 carbon atoms, a di-alkylamino group having 1 to 6 carbon atoms in which alkyl groups are the same or different, a thiol group, an alkylthio group having 1 to 6 carbon atoms, a carboxyl group, an alkoxycarbonyl group having 1 to 6 carbon atoms, and a carbamoyl group. Among these groups, as the linker, a group having a functional group such as a hydroxyl group, an amino group, a thiol group, or a carboxyl group as a substituent is preferable.

Preferred specific examples of the leaving group of R⁶ include a group selected from the following formula (4):

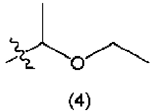

Preferred specific examples of the linker of R⁶ include a group selected from the following formulas (5) to (7):

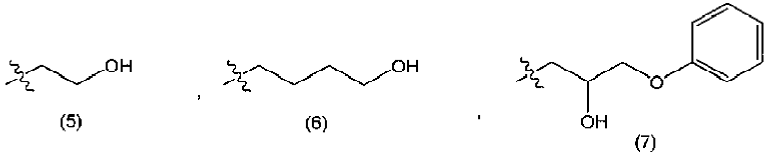

The copolymer of the present invention is a copolymer having structural units represented by formulas (A), (B), and (C). The copolymer may be a random copolymer or a block copolymer, and is preferably a random copolymer. With regard to a composition ratio of each structural unit in one molecule, it is preferable that when (A) is 1 part by mass, (B) is 0.01 to 100 parts by mass and (C) is 0.1 to 100 parts by mass, it is more preferable that when (A) is 1 part by mass, (B) is 0.05 to 18 parts by mass and (C) is 0.1 to 20 parts by mass, and it is particularly preferable that when (A) is 1 part by mass, (B) is 0.7 to 0.9 parts by mass and (C) is 0.1 to 0.3 parts by mass.

A polymerization degree of the copolymer of the present invention is not particularly limited, and is preferably 5 000 to 150 000, and more preferably 8 000 to 150 000 as a number average molecular weight.

In the copolymer of the present invention, as described above, the monomer represented by general formula (1) functions as a unit that imparts hydrophilicity, and the monomer represented by general formula (2) functions as a unit that imparts hydrophobicity. In addition, the monomer represented by general formula (3) functions as a scaffold to which a drug and the copolymer are bound. Examples of the monomer functioning as a hydrophobic unit represented by general formula (2) can include monomers represented by the following formulas:

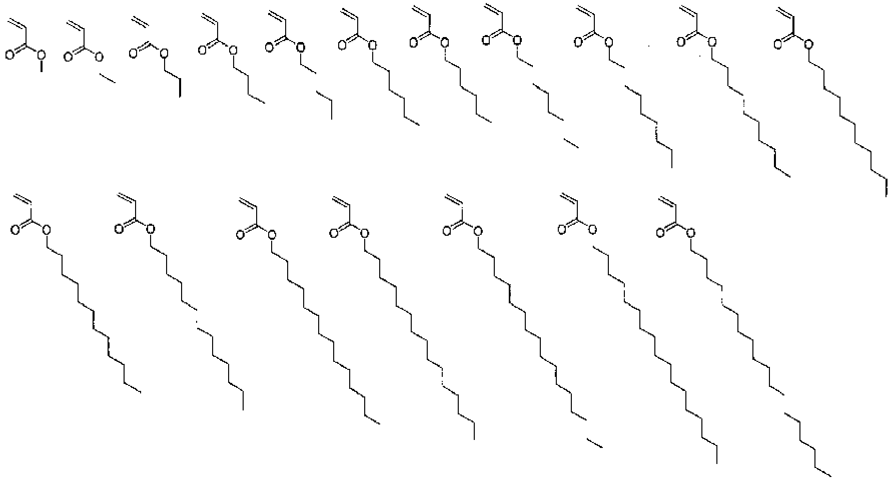

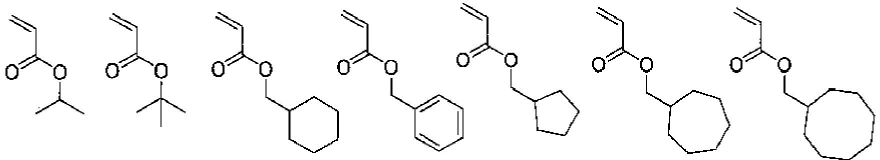

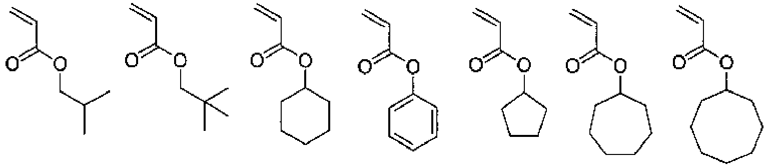

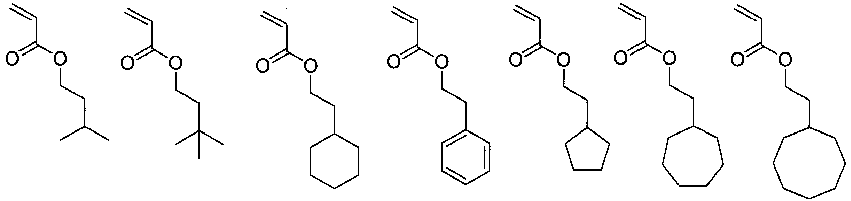

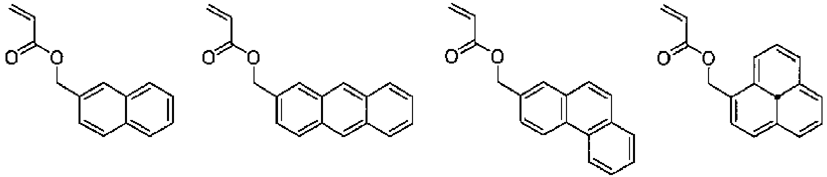

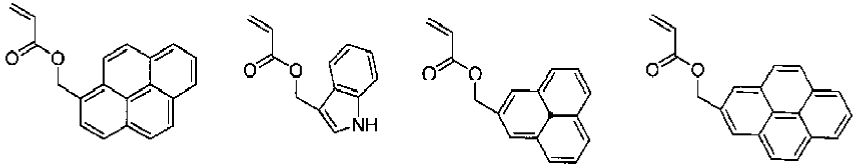

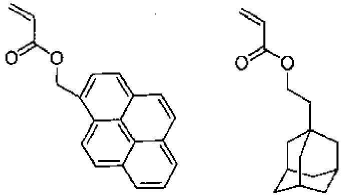

In general formula (1), R¹ represents a hydrogen atom or a C₁₋₃ alkyl group, and is preferably a hydrogen atom or a methyl group, and is preferably a hydrogen atom, an ethyl group, an n-propyl group, or an isopropyl group, and more preferably a hydrogen atom.

In general formula (2), R² represents a hydrogen atom or a C₁₋₃ alkyl group, and is preferably a hydrogen atom or a methyl group, and is preferably a hydrogen atom, an ethyl group, an n-propyl group, or an isopropyl group, and more preferably a hydrogen atom.

In general formula (3), R³ represents a hydrogen atom or a C₁₋₃ alkyl group, and is preferably a hydrogen atom or a methyl group, and is preferably a hydrogen atom, an ethyl group, an n-propyl group, or an isopropyl group, and more preferably a hydrogen atom.

In general formula (1), R⁴ represents a C₁₋₃ alkyl group, specifically a methyl group, an ethyl group, an n-propyl group, or an isopropyl group, preferably a methyl group or an ethyl group, and more preferably a methyl group.

In general formula (1), X¹ represents an oxygen atom, a sulfur atom, or N-R⁷, and is preferably an oxygen atom, a sulfur atom, or NH, and more preferably an oxygen atom.

In general formula (1), m represents an integer of 1 to 100, and is preferably an integer of 3 to 100, and is preferably 3 to 80, more preferably 4 to 60, still more preferably 4 to 40, and yet more preferably 4 to 22 from the viewpoint of imparting good hydrophilicity.

In general formula (2), R⁵ represents a hydrogen atom, a C₁₋₁₈ alkyl group, a 3- to 8-membered cycloalkyl group optionally having a substituent, an adamantyl group, a C₆₋₁₈ aryl group optionally having a substituent, or a 5- to 10-membered heteroaryl group optionally having a substituent, and from the viewpoint of imparting hydrophobicity to the structural unit (B), a C₁₋₁₈ alkyl group, a 3- to 8-membered cycloalkyl group optionally having a substituent, an adamantyl group, a C₆₋₁₈ aryl group optionally having a substituent, or a 5- to 10-membered heteroaryl group optionally having a substituent is preferable, a C₁₋₁₈ alkyl group, a 3- to 8-membered cycloalkyl group optionally having a substituent, an adamantyl group, a C₆₋₁₈ aryl group optionally having a substituent, or a 5- to 10-membered heteroaryl group optionally having a substituent is more preferable, and a C₁₋₁₈ alkyl group, a 3- to 8-membered cycloalkyl group, an adamantyl group, or a C₆₋₁₈ aryl group is still more preferable. In addition, a 3- to 8-membered cycloalkyl group optionally having a substituent, an adamantyl group, a C₆₋₁₄ aryl group optionally having a substituent, or a 6- to 10-membered heteroaryl group optionally having a substituent is also preferable. Here, the substituent is preferably one or two or more selected from a halogen atom, an alkyl group having 1 to 6 carbon atoms, an alkenyl group having 2 to 6 carbon atoms, and an alkynyl group having 2 to 6 carbon atoms.

In general formula (2), X² represents an oxygen atom, a sulfur atom, or N-R⁷, and is preferably an oxygen atom, a sulfur atom, or NH, and more preferably an oxygen atom.

In general formula (2), n represents an integer of 0 to 3, preferably an integer of 1 to 3, and more preferably 1.

In general formula (3), R⁶ represents a hydrogen atom, a leaving group, or a linker. As the leaving group or linker, a C₁₋₁₈ alkyl group optionally having a substituent, a 3- to 8-membered cycloalkyl group optionally having a substituent, or a C₇₋₁₉ aralkyl group optionally having a substituent is preferable. Here, examples of the substituent include a halogen atom, an alkyl group having 1 to 6 carbon atoms, an alkenyl group having 2 to 6 carbon atoms, an alkynyl group having 2 to 6 carbon atoms, a hydroxyl group, an alkoxy group having 1 to 6 carbon atoms, an amino group, an alkylamino group having 1 to 6 carbon atoms, a di-alkylamino group having 1 to 6 carbon atoms in which alkyl groups are the same or different, a thiol group, an alkylthio group having 1 to 6 carbon atoms, a carboxyl group, an alkoxycarbonyl group having 1 to 6 carbon atoms, and a carbamoyl group. Among these groups, as the linker, a group having a functional group such as a hydroxyl group, an amino group, a thiol group, or a carboxyl group as a substituent is preferable.

Preferred specific examples of the leaving group of R⁶ include a group selected from the following formula (4):

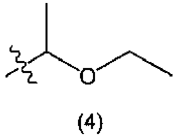

Preferred specific examples of the linker of R⁶ include a group selected from the following formulas (5) to (7):

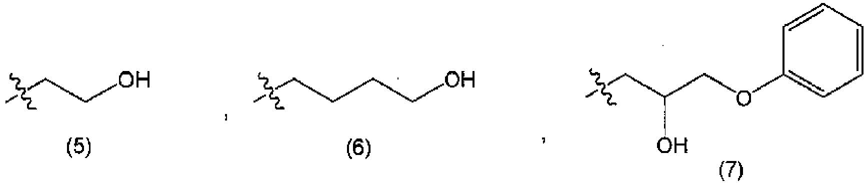

In general formula (3), X³ represents an oxygen atom, a sulfur atom, or N-R⁷, and is preferably an oxygen atom, a sulfur atom, or NH, and more preferably an oxygen atom.

The copolymer of the present invention is formed by copolymerizing three monomers represented by general formulas (1) to (3). The copolymerization may be random copolymerization or block copolymerization, and those formed by random copolymerization are preferable. As for a blending ratio of the three monomers, it is preferable that 0.01 to 100 parts by mass of monomer (2) and 0.1 to 100 parts by mass of monomer (3) are polymerized, it is more preferable that 0.05 to 18 parts by mass of monomer (2) and 0.1 to 20 parts by mass of monomer (3) are polymerized, and it is particularly preferable that 0.7 to 0.9 parts by mass of monomer (2) and 0.1 to 0.3 parts by mass of monomer (3) are polymerized, with respect to 1 part by mass of monomer (1) .

In addition, "solvates" in which various solvents are coordinated are also included in the copolymer of the present invention. In the present description, examples of the "solvate" include a hydrate and an ethanolate. The solvent may be coordinated to the copolymer of the present invention in any number.

In the present description, the term "pharmaceutical composition" means one in which active ingredients (drug, physiologically active substance) that can be used for diagnosis, prevention, or treatment of a disease are supported on the copolymer of the present invention by an action such as electrostatic interaction, hydrogen bonding, hydrophobic interaction, or covalent bonding. When the copolymer forms nanoparticles, examples thereof include a form in which the drug is present on the particle surface, a form in which the drug is encapsulated in the nanoparticle, and a combination form thereof.

The copolymer of the present invention can be produced by various known methods. The production method is not particularly limited, and for example, the copolymer can be produced according to a basic polymer synthesis method described below. 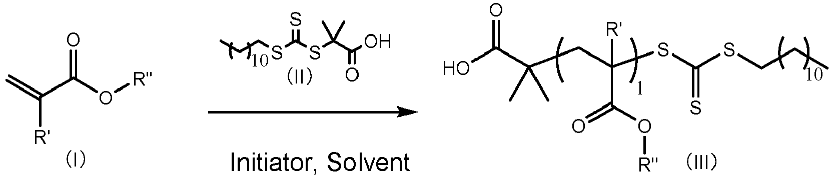 wherein R' represents a hydrogen atom or a C₁₋₃ alkyl group, and R" represents the group represented by R⁴, R⁵, or R⁶.

This reaction shows a step of producing a polymer (III) by reacting a monomer (I) with a chain transfer agent (II) and an initiator. This reaction can be performed in the absence of a solvent or in a solvent such as alcohols such as methanol, ethanol, 1-propanol, and 2-propanol; ethers such as diethyl ether, tetrahydrofuran, and 1,4-dioxane; aromatic hydrocarbons such as benzene, toluene, and xylene; halogenated hydrocarbons such as dichloromethane, chloroform, and 1,2-dichloroethane; N,N-dimethylformamide; N,N-dimethylacetamide; N-methylpyrrolidone; acetonitrile; and ethyl acetate, and it is preferable to use aromatic hydrocarbons such as toluene and xylene as a solvent. As the chain transfer agent, for example, 2-(dodecylthiocarbonothioylthio)-2-methylpropionic acid (DDMAT), cyanomethyl dodecyltrithiocarbonate (CDTTC), 2-cyano-2-propyldodecyl trithiocarbonate (CPDTTC), or 4-cyano-4-[(dodecylsulfanyl-thiocarbonyl)sulfanyl]pentanoic acid (CDSPA) can be used, and DDMAT is preferably used. When polymerized using the chain transfer agent, the copolymer of the present invention has a structure in which a part or all of the structure of the chain transfer agent is partially bound. When the copolymer contains the structure of the serial transfer agent, the structure may be removed by an appropriate method. As the initiator, azo polymerization initiators such as 2,2'-azobisisobutyronitrile (AIBN), 1,1'-azobis(cyclohexanecarbonitrile) (ACHN), 2,2'-azobis-2-methylbutyronitrile (AMBN), and 2,2'-azobis-2,4-dimethylvaleronitrile (ADVN) can be used, and AIBN is preferably used. The reaction temperature is 0 to 300°C, preferably 0 to 150°C, more preferably room temperature to 100°C, and reaction time is 1 minute to 48 hours, and preferably 5 minutes to 24 hours. In this reaction, a random copolymerized copolymer can be produced by carrying out the reaction in the coexistence of monomers (I) having different structures.

The produced copolymer of the present invention can be purified by a polymer isolation and purification method generally known in the field of polymer chemistry. Specific examples thereof include treatment operations such as extraction, recrystallization, salting out using, for example, ammonium sulfate or sodium sulfate, centrifugation, dialysis, ultrafiltration, adsorption chromatography, ion exchange chromatography, hydrophobic chromatography, normal phase chromatography, reverse phase chromatography, gel filtration, gel permeation chromatography, affinity chromatography, electrophoresis, countercurrent distribution, and combinations thereof.

The copolymer of the present invention can be used as a carrier for transportation of various physiologically active substances (drugs). For example, a pharmaceutical composition in which a therapeutic agent for tumor is supported on (encapsulated in) the copolymer of the present invention can be used as a prophylactic and/or therapeutic agent for various cancer diseases such as colon cancer, duodenal cancer, gastric cancer, pancreatic cancer, liver cancer, lung cancer, uterine cancer, and ovarian cancer, for example, because the pharmaceutical composition suppresses growth of a tumor as confirmed in Test Examples described below. In addition, since the pharmaceutical composition has a high tumor accumulation ability, it can be used as a diagnostic agent or contrast agent for a tumor.

When the copolymer of the present invention is used as a drug transport carrier, the dose and the number of doses may be appropriately selected in consideration of, for example, administration form, age and body weight of a patient, and nature or severity of a symptom to be treated, and the dose and the number of doses should not be limited. However, when a polymer encapsulating a drug is intravenously injected by an injection, for example, an amount of 0.12 mg to 12 000 000 mg is preferably administered, an amount of 1.2 mg to 1 200 000 mg is more preferably administered, and an amount of 12 to 120 000 mg is particularly preferably administered per adult (60 kg).

The pharmaceutical composition of the present invention can be produced by mixing the copolymer of the present invention and a drug. Preferably, the copolymer of the present invention and a drug may be mixed to produce a single chain nanoparticle, or a single chain nanoparticle of the copolymer of the present invention may be produced and then a drug may be mixed. The single chain nanoparticle can be produced by a known method.

In the pharmaceutical composition of the present invention, the drug may be supported on the copolymer by an action such as electrostatic interaction, hydrogen bonding, hydrophobic interaction, or covalent bonding.

Examples of the drug used for various cancer diseases include pemetrexed sodium, mitomycin C, bleomycin hydrochloride, aclarubicin hydrochloride, amrubicin hydrochloride, epirubicin hydrochloride, doxorubicin hydrochloride, pirarubicin hydrochloride, irinotecan hydrochloride, etoposide, docetaxel, nogitecan hydrochloride, paclitaxel, vinorelbine tartrate, vinblastine sulfate, oxaliplatin, carfilzomib, carboplatin, cisplatin, temsirolimus, trabectedin, fulvestrant, bortezomib, mitoxantrone hydrochloride, miriplatin, emtansine, SN-38, monomethyl auristatin E, monomethyl auristatin F, and staurosporine. When these drugs are prepared in the pharmaceutical composition of the present invention, the drugs may be supported on the copolymer as a free form.

A route of administration of the pharmaceutical composition of the present invention is desirably the most effective one for treatment, and the pharmaceutical composition can be administered by a parenteral administration preparation such as an oral administration preparation, an injection, or a transdermal administration preparation. For example, parenteral administration such as intraarterial injection, intravenous injection, subcutaneous injection, intramuscular injection, or intraperitoneal injection is preferable, and intraarterial injection and intravenous injection are more preferable. The number of doses should not be limited, and examples thereof include one to several doses per week average.

Various preparations suitable for the route of administration can be produced by a conventional method by appropriately selecting preparation additives such as excipients, extenders, binders, wetting agents, disintegrants, lubricants, surfactants, dispersants, buffers, preservatives, solubilizing agents, antiseptics, flavoring agents, soothing agents, stabilizers, and isotonizing agents that are usually used in formulation.

The preparation additives that can be contained in the various preparations described above are not particularly limited as long as the preparation additives are pharmaceutically acceptable. Examples of such preparation additives include purified water, water for injection, distilled water for injection, pharmaceutically acceptable organic solvents, collagen, polyvinyl alcohol, polyvinylpyrrolidone, carboxyvinyl polymer, sodium alginate, water-soluble dextran, sodium carboxymethyl starch, pectin, xanthan gum, gum arabic, casein, gelatin, agar, glycerin, propylene glycol, polyethylene glycol, petrolatum, paraffin, stearyl alcohol, stearic acid, human serum albumin, mannitol, sorbitol, and lactose. Additives to be used are appropriately selected according to various preparations, and can be used alone or in combination.

The injection can also be prepared as a non-aqueous diluent (for example, polyethylene glycol, vegetable oils such as olive oil, and alcohols such as ethanol), a suspension, or an emulsion. Sterilization of the injection can be performed by filtration sterilization using a filter, and blending of, for example, a microbicide. In addition, the injection can be produced in a form of preparation before use. That is, the injection can be formed into a sterile solid composition by, for example, a freeze-drying method, and can be dissolved in water for injection, distilled water for injection, or another solvent before use.

### Examples

Hereinafter, the present invention will be described more specifically with reference to Examples. These Examples are provided for purposes of illustration and are not intended to limit embodiments of the present invention.

### [Example 1] Production of poly[(benzyl acrylate)-co-(poly(ethylene glycol) methyl ether acrylate)-co-(1-ethoxyethyl acrylate)]

### (1) Synthesis of 1-ethoxyethyl acrylate (EEA)

Ethyl vinyl ether (28.725 mL) was weighed under an argon atmosphere, and phosphoric acid (50 mg) was added thereto under ice cooling. Thereafter, acrylic acid (17.15 mL) was added thereto, and the mixture was stirred at room temperature for 48 hours. Hydrotalcite (3 g) was added thereto, and the mixture was further stirred for 2 hours to stop the reaction. After Celite filtration, unreacted ethyl vinyl ether was removed by evaporation. Phenothiazine as a polymerization inhibitor was added so as to be 500 ppm, and the mixture was purified by distillation under reduced pressure together with calcium hydride (distillation temperature: 28 to 32°C). The obtained 1-ethoxyethyl acrylate was separated into glass vials and stored at -30°C.

¹³C NMR (400MHz, CDCl₃), δ, ppm: 15.29 (-OCH₂CH₃), 21.16 (-COOCH(CH₃)), 64.98 (-OCH₂-), 96.73 (-COOCH(CH₃)), 128.84 (CH₂CH-), 131.43 (CH₂CH-), 166.00 (-COO).

### (2) Synthesis of poly[(benzyl acrylate)-co-(poly(ethylene glycol) methyl ether acrylate)-co-(1-ethoxyethyl acrylate)]

100 mg of 2-(dodecylthiocarbonothioylthio)-2-methylpropionic acid (DDMAT) was weighed and dissolved in 17.3 mL of toluene to prepare a DDMAT/toluene stock solution (5.78 mg/mL as a DDMAT concentration). Similarly, 22 mg of 2,2'-azobis(2-methylpropionitrile) (AIBN) was weighed and dissolved in 17.3 mL of toluene to prepare an AIBN/toluene stock solution (AIBN concentration: 1.27 mg/mL). Separately, 1.296 g of poly(ethylene glycol) methyl ether acrylate (mPEGA, the average value (n) of the numbers of repetitions of ethylene glycol is 9), 0.394 g of benzyl acrylate (BnA), 0.039 g of 1-ethoxyethyl acrylate, 1.73 mL of a DDMAT/toluene stock solution, and 1.73 mL of an AIBN/toluene stock solution were added, and polymerization was performed in an oil bath at 70°C. After a lapse of 90 minutes, the polymerization was stopped, and then the reaction solution was subjected to a reprecipitation method or dialyzed against methanol to recover the copolymer. Since the obtained copolymer was basically a viscous body, in the reprecipitation method, an operation of dropping the reaction solution into a centrifuge tube to which a poor solvent (hexane/ethyl acetate = 7/3 [v/v]) was added and recovering the solution by centrifugation (2 000 × g, 5 min) was repeated 3 times, and finally vacuum drying was performed to obtain 1.223 g of poly[(benzyl acrylate)-co-(poly(ethylene glycol) methyl ether acrylate)-co-(1-ethoxyethyl acrylate)].

As a result of analyzing the polymerization degree of each monomer and the number average molecular weight (M_{n,NMR}) from a ¹H-NMR spectrum of the obtained copolymer measured using NMR, the polymerization degree of mPEGA (n = 9) was 102, the polymerization degree of BnA was 94, the polymerization degree of EEA was 9, and M_{n,NMR} was 65,900. The molecular weight dispersion (M_{w}/Mₙ) of the obtained copolymer was measured using GPC, and as a result, it was found to be 1.53.

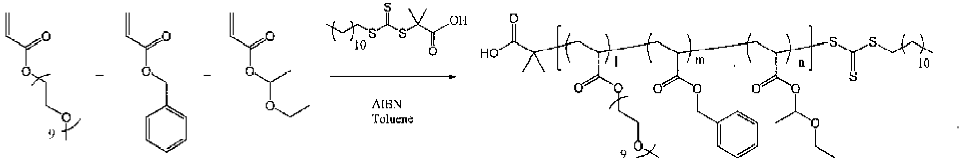

### [Measurement apparatus and conditions]

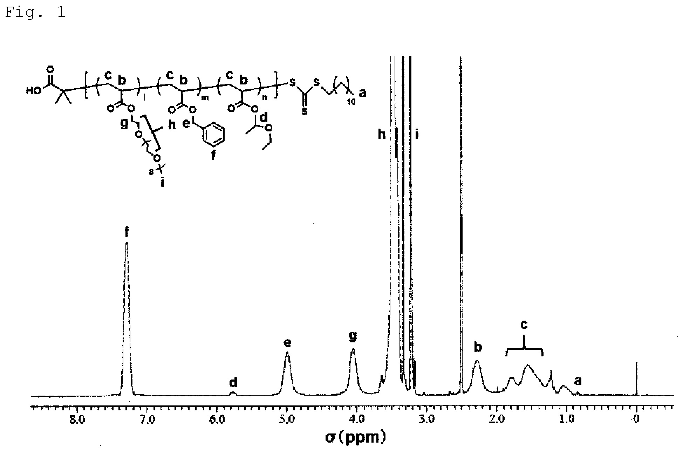

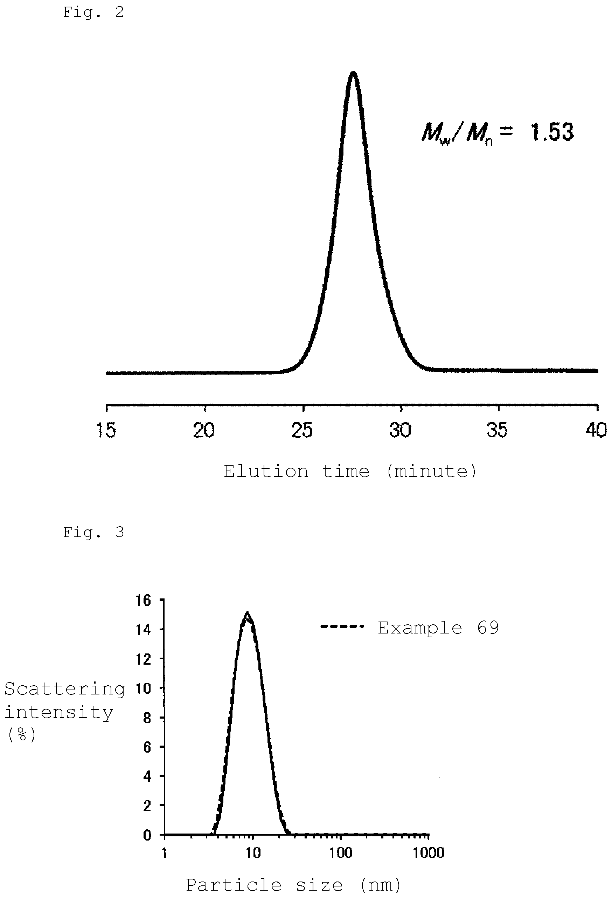

(1) ¹H-NMR measurement
   Apparatus: JNM-ECX 400 (400 MHz)/JEOL Ltd.
   Solvent: Dimethyl sulfoxide-d₆ containing 0.03% tetramethylsilane/KANTO CHEMICAL CO., INC.
   Sample concentration: 20 mg/mL
   Measurement temperature: 25°C
   Cumulative number: 256 times
   Result: Fig. 1
(2) GPC measurement
   Apparatus: HPLC-Prominence system/Shimadzu Corporation
   Detector: RID-10A refractive index detector/Shimadzu Corporation
   Column: TSKgel α-2500 column/Tosoh Corporation
   (Column size: 7.8 mm × 300 mm, particle size: 7 um, exclusion limit molecular weight: 5 × 10³)
   TSKgel α-4000 column/Tosoh Corporation
   (Column size: 7.8 mm × 300 mm, particle size: 10 um, exclusion limit molecular weight: 4 × 10⁵)
   TSKgel guardcolum/Tosoh Corporation
   Mobile phase: N,N-dimethyformamide (DMF) containing 10 mmol/L lithium bromide
   Temperature: 40°C
   Flow rate: 0.5 mL/min
   Sample concentration: 6 mg/mL
   Standard substance: Poly(methyl methacrylate) standard ReadyCal set, Mₚ 800 to 2 200 000 Da/SIGMA
   Result: Fig. 2

**[Table 1]**

| Example | Charge ratio (molar ratio to chain transfer agent) | | | | | Solvent | Temperature (°C) | Polymerization time (min) | Yield (g) | Polymerization degree | | | *M*_{n,NMR} | *M_{w}*/*Mₙ* |
|---|---|---|---|---|---|---|---|---|---|---|---|---|---|---|
| | Chain transfer agent | Initiator | Monomer | | | | | | | | | | | |
| | DDMAT | AIBN | mPEGA | BnA | EEA | | | | | mPE GA | BnA | EEA | | |
| | | | n=9 | | | | | | | n=9 | | | | |
| 1 | 1 | 0.5 | 100 | 90 | 10 | Toluene | 70 | 90 | 1.223 | 102 | 94 | 9 | 65,900 | 1.53 |

### [Examples 2 to 68]

Polymers having different composition ratios and average molecular weights shown in the following table were produced by appropriately changing the type, charged amount, reaction temperature, and polymerization time of the monomers (mPEGA, BnA, and EEA) used in Example 1, and using the same method as in Example 1.

**[Table 2]**

| Example | Charge ratio (molar ratio to chain transfer agent) | | | | | | | | | | | Solvent | Temperature (°C) | Polymerization time (min) |
|---|---|---|---|---|---|---|---|---|---|---|---|---|---|---|
| | Chain transfer agent | Initiator | | Monomer | | | | | | | | | | |
| | DDMAT | AIBN | ACHN | mPEGA | | | BnA | EEA | 2-Hydroxy ethyl acrylate | 4-Hydroxy butyl acrylate | 2-Hydroxy-3-phenoxy propyl acrylate | | | |
| | | | | n=4 | n=9 | n=22 | | | | | | | | |
| 2 | 1 | 2 | - | 255 | - | - | 15 | 30 | - | - | - | Toluene | 70 | 90 |
| 3 | 1 | 2 | - | 160 | - | - | 20 | 20 | - | - | - | Toluene | 70 | 60 |
| 4 | 1 | 2 | - | 240 | - | - | 30 | 30 | - | - | - | Toluene | 70 | 60 |
| 5 | 1 | 2 | - | 170 | - | - | 10 | 20 | - | - | - | Toluene | 70 | 60 |
| 6 | 1 | 2 | - | 255 | - | - | 15 | 30 | - | - | - | Toluene | 70 | 60 |
| 7 | 1 | 0.5 | - | - | 16 | - | 16 | 8 | - | - | - | Toluene | 70 | 90 |
| 8 | 1 | 0.5 | - | - | 18 | - | 14 | 8 | - | - | - | Toluene | 70 | 90 |
| 9 | 1 | 0.5 | - | - | 20 | - | 12 | 8 | - | - | - | Toluene | 70 | 90 |
| 10 | 1 | 0.5 | - | - | 20 | - | 12 | 8 | - | - | - | Toluene | 70 | 90 |
| 11 | 1 | 0.5 | - | - | 20 | - | 12 | 8 | - | - | - | Toluene | 70 | 90 |
| 12 | 1 | 0.5 | - | - | 20 | - | 16 | 4 | - | - | - | Toluene | 70 | 10 |
| 13 | 1 | 0.5 | - | - | 20 | - | 16 | 4 | - | - | - | Toluene | 70 | 30 |
| 14 | 1 | 0.5 | - | - | 20 | - | 16 | 4 | - | - | - | Toluene | 70 | 50 |
| 15 | 1 | 0.5 | - | - | 20 | - | 16 | 4 | - | - | - | Toluene | 70 | 70 |
| 16 | 1 | 0.5 | - | - | 20 | - | 16 | 4 | - | - | - | Toluene | 70 | 90 |
| 17 | 1 | 0.5 | - | - | 22 | - | 10 | 8 | - | - | - | Toluene | 70 | 90 |
| 18 | 1 | 0.5 | - | - | 24 | - | 8 | 8 | - | - | - | Toluene | 70 | 90 |
| 19 | 1 | 0.5 | - | - | 24 | - | 8 | 8 | - | - | - | Toluene | 70 | 90 |
| 20 | 1 | 0.5 | - | - | 28 | - | 4 | 8 | - | - | - | Toluene | 70 | 90 |
| 21 | 1 | 0.5 | - | - | 10 | - | 25 | 15 | - | - | - | Toluene | 70 | 90 |
| 22 | 1 | 0.5 | - | - | 17 | - | 25 | 8 | - | - | - | Toluene | 70 | 90 |
| 23 | 1 | 0.5 | - | - | 22.5 | - | 12.5 | 15 | - | - | - | Toluene | 70 | 90 |
| 24 | 1 | 0.5 | - | - | 25 | - | 20 | 5 | - | - | - | Toluene | 70 | 90 |
| 25 | 1 | 0.5 | - | - | 29.5 | - | 12.5 | 8 | - | - | - | Toluene | 70 | 90 |
| 26 | 1 | 0.5 | - | - | 30 | - | 24 | 6 | - | - | - | Toluene | 70 | 90 |
| 27 | 1 | 0.5 | - | - | 35 | - | 28 | 7 | - | - | - | Toluene | 70 | 90 |
| 28 | 1 | 0.5 | - | - | 50 | - | 40 | 10 | - | - | - | Toluene | 70 | 90 |
| 29 | 1 | 0.5 | - | - | 10 | - | 10 | 180 | - | - | - | Toluene | 70 | 90 |
| 30 | 1 | 0.5 | - | - | 30 | - | 30 | 140 | - | - | - | Toluene | 70 | 90 |
| 31 | 1 | 0.5 | - | - | 30 | - | 70 | 100 | - | - | - | Toluene | 70 | 90 |
| 32 | 1 | 0.5 | - | - | 30 | - | 110 | 60 | - | - | - | Toluene | 70 | 90 |
| 33 | 1 | 0.5 | - | - | 50 | - | 10 | 140 | - | - | - | Toluene | 70 | 90 |
| 34 | 1 | 0.5 | - | - | 50 | - | 50 | 100 | - | - | - | Toluene | 70 | 90 |
| 35 | 1 | 0.5 | - | - | 50 | - | 90 | 60 | - | - | - | Toluene | 70 | 90 |
| 36 | 1 | 0.5 | - | - | 50 | - | 130 | 20 | - | - | - | Toluene | 70 | 90 |
| 37 | 1 | 0.5 | - | - | 70 | - | 30 | 100 | - | - | - | Toluene | 70 | 90 |
| 38 | 1 | 0.5 | - | - | 70 | - | 70 | 60 | - | - | - | Toluene | 70 | 90 |
| 39 | 1 | 0.5 | - | - | 70 | - | 110 | 20 | - | - | - | Toluene | 70 | 90 |
| 40 | 1 | 0.5 | - | - | 80 | - | 80 | 40 | - | - | - | Toluene | 70 | 90 |
| 41 | 1 | 0.5 | - | - | 80 | - | 100 | 20 | - | - | - | Toluene | 70 | 90 |
| 42 | 1 | 0.5 | - | - | 90 | - | 10 | 100 | - | - | - | Toluene | 70 | 90 |
| 43 | 1 | 0.5 | - | - | 90 | - | 50 | 60 | - | - | - | Toluene | 70 | 90 |
| 44 | 1 | 0.5 | - | - | 90 | - | 90 | 20 | - | - | - | Toluene | 70 | 90 |
| 45 | 1 | 0.5 | - | - | 100 | - | 20 | 80 | - | - | - | Toluene | 70 | 90 |
| 46 | 1 | 0.5 | - | - | 100 | - | 40 | 60 | - | - | - | Toluene | 70 | 90 |
| 47 | 1 | 0.5 | - | - | 100 | - | 50 | 50 | - | - | - | Toluene | 70 | 90 |
| 48 | 1 | 0.5 | - | - | 100 | - | 60 | 40 | - | - | - | Toluene | 70 | 90 |
| 49 | 1 | 0.5 | - | - | 100 | - | 70 | 30 | - | - | - | Toluene | 70 | 90 |
| 50 | 1 | 0.5 | - | - | 100 | - | 80 | 20 | - | - | - | Toluene | 70 | 90 |
| 51 | 1 | 0.5 | - | - | 110 | - | 30 | 60 | - | - | - | Toluene | 70 | 90 |
| 52 | 1 | 0.5 | - | - | 110 | - | 70 | 20 | - | - | - | Toluene | 70 | 90 |
| 53 | 1 | 0.5 | - | - | 130 | - | 10 | 60 | - | - | - | Toluene | 70 | 90 |
| 54 | 1 | 0.5 | - | - | 130 | - | 50 | 20 | - | - | - | Toluene | 70 | 90 |
| 55 | 1 | 0.5 | - | - | 150 | - | 30 | 20 | - | - | - | Toluene | 70 | 90 |
| 56 | 1 | 0.5 | - | - | 170 | - | 10 | 20 | - | - | - | Toluene | 70 | 90 |
| 57 | 1 | 0.5 | - | - | 200 | - | 160 | 40 | - | - | - | Toluene | 70 | 90 |
| 58 | 1 | 0.5 | - | - | 300 | - | 240 | 60 | - | - | - | Toluene | 70 | 90 |
| 59 | 1 | 0.5 | - | - | 400 | - | 320 | 80 | - | - | - | Toluene | 70 | 90 |
| 60 | 1 | 2 | - | - | - | 105 | 155 | 40 | - | - | - | Toluene | 70 | 90 |
| 61 | 1 | 2 | - | - | - | 120 | 240 | 40 | - | - | - | Toluene | 70 | 90 |
| 62 | 1 | 2 | - | - | - | 140 | 210 | 50 | - | - | - | Toluene | 70 | 90 |
| 63 | 1 | 2 | - | - | - | 140 | 220 | 40 | - | - | - | Toluene | 70 | 90 |
| 64 | 1 | 0.1 | - | - | 100 | - | 80 | - | 20 | - | - | 1,4-Dioxane | 70 | 90 |
| 65 | 1 | - | 0.5 | - | 100 | - | 50 | - | - | 50 | - | 1,4-Dioxane | 70 | 180 |
| 66 | 1 | - | 0.5 | - | 100 | - | 80 | - | - | 20 | - | 1,4-Dioxane | 70 | 180 |
| 67 | 1 | 0.1 | - | - | 130 | - | 65 | - | - | - | 65 | 1 ,4-Dioxane | 70 | 90 |
| 68 | 1 | 0.1 | - | - | 130 | - | 104 | - | - | - | 26 | 1 ,4-Dioxane | 70 | 90 |

**[Table 3]**

| Example | Polymerization degree | | | | | | | | *M*_{n,NMR} | *M*_{w}/*M*ₙ |
|---|---|---|---|---|---|---|---|---|---|---|
| | mPEGA | | | BnA | EEA | 2-Hydroxy ethyl acrylate | 4-Hydroxy butyl acrylate | 2-Hydroxy-3-phenoxy propyl acrylate | | |
| | n=4 | n=9 | n=22 | | | | | | | |
| 2 | 250 | - | - | 18 | 31 | - | - | - | 62 400 | 1.37 |
| 3 | 155 | - | - | 22 | 20 | - | - | - | 39 200 | 123 |
| 4 | 211 | - | - | 29 | 29 | - | - | - | 53 100 | 132 |
| 5 | 162 | - | - | 11 | 20 | - | - | - | 39 000 | 122 |
| 6 | 227 | - | - | 15 | 28 | - | - | - | 54 300 | 1.33 |
| 7 | - | 16 | - | 16 | 8 | - | - | - | 11 800 | 120 |
| 8 | - | 18 | - | 14 | 8 | - | - | - | 12 400 | 120 |
| 9 | - | 13 | - | 8 | 5 | - | - | - | 8 600 | 1.46 |
| 10 | - | 20 | - | 13 | 8 | - | - | - | 13 400 | 130 |
| 11 | - | 18 | - | 11 | 7 | - | - | - | 11 700 | 1.33 |
| 12 | - | 8 | - | 7 | 1 | - | - | - | 5 300 | 1.51 |
| 13 | - | 16 | - | 13 | 3 | - | - | - | 10 800 | 130 |
| 14 | - | 19 | - | 15 | 3 | - | - | - | 12 200 | 1.28 |
| 15 | - | 20 | - | 16 | 4 | - | - | - | 13 300 | 1.39 |
| 16 | - | 24 | - | 19 | 4 | - | - | - | 15 700 | 1.29 |
| 17 | - | 21 | - | 10 | 8 | - | - | - | 13 200 | 120 |
| 18 | - | 13 | - | 5 | 4 | - | - | - | 8 000 | 1.44 |
| 19 | - | 24 | - | 8 | 8 | - | - | - | 14 300 | 121 |
| 20 | - | 26 | - | 4 | 8 | - | - | - | 14 600 | 120 |
| 21 | - | 7 | - | 17 | 9 | - | - | - | 8 100 | 1.41 |
| 22 | - | 11 | - | 17 | 5 | - | - | - | 9200 | 1.39 |
| 23 | - | 15 | - | 9 | 10 | - | - | - | 10 700 | 130 |
| 24 | - | 26 | - | 21 | 5 | - | - | - | 16 900 | 1.31 |
| 25 | - | 20 | - | 10 | 6 | - | - | - | 12 400 | 1.33 |
| 26 | - | 33 | - | 27 | 6 | - | - | - | 21 500 | 1.34 |
| 27 | - | 39 | - | 30 | 7 | - | - | - | 25000 | 1.36 |
| 28 | - | 39 | - | 4 | 8 | - | - | - | 20 200 | 125 |
| 29 | - | 10 | - | 10 | 159 | - | - | - | 29 700 | 1.19 |
| 30 | - | 27 | - | 27 | 122 | - | - | - | 35600 | 1.27 |
| 31 | - | 30 | - | 66 | 91 | - | - | - | 38 600 | 1.17 |
| 32 | - | 27 | - | 91 | 52 | - | - | - | 35 800 | 120 |
| 33 | - | 45 | - | 10 | 123 | - | - | - | 41 000 | 1.31 |
| 34 | - | 44 | - | 45 | 86 | - | - | - | 41 100 | 120 |
| 35 | - | 45 | - | 79 | 54 | - | - | - | 42 700 | 124 |
| 36 | - | 43 | - | 111 | 19 | - | - | - | 42 000 | 128 |
| 37 | - | 60 | - | 28 | 87 | - | - | - | 46 500 | 124 |
| 38 | - | 55 | - | 57 | 49 | - | - | - | 43 000 | 1.27 |
| 39 | - | 56 | - | 89 | 18 | - | - | - | 44 300 | 130 |
| 40 | - | 79 | - | 80 | 36 | - | - | - | 56 400 | 143 |
| 41 | - | 81 | - | 106 | 17 | - | - | - | 58 900 | 1.51 |
| 42 | - | 81 | - | 12 | 93 | - | - | - | 54600 | 1.27 |
| 43 | - | 71 | - | 42 | 51 | - | - | - | 48 600 | 130 |
| 44 | - | 70 | - | 73 | 18 | - | - | - | 48 400 | 132 |
| 45 | - | 84 | - | 19 | 72 | - | - | - | 54 200 | 133 |
| 46 | - | 80 | - | 35 | 52 | - | - | - | 51 900 | 1.35 |
| 47 | - | 84 | - | 45 | 44 | - | - | - | 54 200 | 133 |
| 48 | - | 82 | - | 52 | 35 | - | - | - | 53 100 | 136 |
| 49 | - | 94 | - | 68 | 26 | - | - | - | 60 100 | 1.50 |
| 50 | - | 104 | - | 83 | 20 | - | - | - | 66 700 | 1.52 |
| 51 | - | 93 | - | 30 | 55 | - | - | - | 57 700 | 133 |
| 52 | - | 84 | - | 56 | 18 | - | - | - | 52 400 | 135 |
| 53 | - | 105 | - | 10 | 52 | - | - | - | 60000 | 135 |
| 54 | - | 102 | - | 42 | 18 | - | - | - | 58 900 | 1.37 |
| 55 | - | 111 | - | 25 | 18 | - | - | - | 60 100 | 140 |
| 56 | - | 131 | - | 10 | 18 | - | - | - | 67 400 | 143 |
| 57 | - | 129 | - | 108 | 31 | - | - | - | 84100 | 1.51 |
| 58 | - | 170 | - | 144 | 40 | - | - | - | 111200 | 161 |
| 59 | - | 221 | - | 191 | 50 | - | - | - | 144 700 | 1.68 |
| 60 | - | - | 58 | 102 | 78 | - | - | - | 91 200 | 1.71 |
| 61 | - | - | 62 | 143 | 21 | - | - | - | 93 100 | 168 |
| 62 | - | - | 59 | 105 | 93 | - | - | - | 95000 | 182 |
| 63 | - | - | 60 | 106 | 18 | - | - | - | 85 400 | 168 |
| 64 | - | 75 | - | 66 | - | 19 | - | - | 49 400 | 1.46 |
| 65 | - | 67 | - | 37 | - | - | 39 | - | 44 300 | 140 |
| 66 | - | 61 | - | 56 | - | - | 20 | - | 41 600 | 143 |
| 67 | - | 75 | - | 42 | - | - | - | 42 | 52 300 | 1.61 |
| 68 | - | 75 | - | 68 | - | - | - | 18 | 51 400 | 1.56 |

### [Example 69]

### Production of poly[(benzyl acrylate)-co-(poly(ethylene glycol) methyl ether acrylate)-co-(acrylic acid)]

By treating the poly[(benzyl acrylate)-co-(poly(ethylene glycol) methyl ether acrylate)-co-(1-ethoxyethyl acrylate)] obtained in Example 1 with 0.5 N HCl at room temperature, the ethoxyethyl group was eliminated to obtain 1.176 g of a terpolymer having a carboxyl group. The Z-average particle size and the polydispersity index of the obtained terpolymer in water were measured by dynamic light scattering (DLS), and as a result, the Z-average particle size was found to be 8.5 nm (polydispersity index: 0.14) .

### [Measurement apparatus and conditions]

### (1) DLS measurement

Apparatus: Zetasizer NanoZS/Malvern Instruments Ltd.
Measurement temperature: 25°C
Sample concentration: 10 mg/mL
Result: Fig. 3

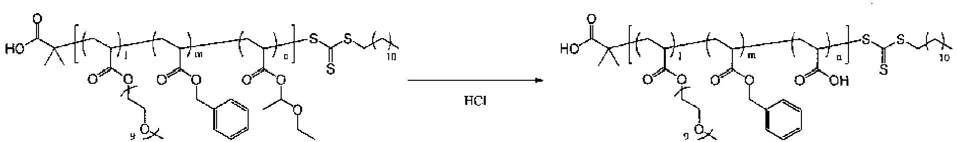

### [Example 70]

### Method for producing (1,2-diaminocyclohexane)platinum(II)-encapsulating SCNP

65.28 mg of a Cl(H₂O) form of (1,2-diaminocyclohexane)platinum(II) (hereinafter, abbreviated as DACHPt) (DACHPt·Cl·H₂O) was dissolved in 20 mL of purified water and stirred at 70°C for 2 hours. To 5 mL of this solution, 287.4 mg of the terpolymer obtained in Example 69 was added, and the mixture was stirred at 50°C overnight. After completion of stirring, the reaction solution was dialyzed and purified using purified water as an external solution to obtain 5 mL of a DACHPt-encapsulating SCNP. The Pt content of the DACHPt-encapsulating SCNP obtained after purification was measured by inductively coupled plasma-atomic emission spectrometry (ICP-AES) and found to be 720 ug/mL (1.14 mg/mL as DACHPt). Separately, 200 µL of the DACHPt-encapsulating SCNP was freeze-dried, and the solid content concentration was calculated, then the ratio to the Pt content was taken, and the Pt binding amount per polymer was calculated. As a result, the Pt binding amount was 3.4 mol/mol. In addition, the Z-average particle size and the polydispersity index of the obtained DACHPt-encapsulating SCNP were measured by dynamic light scattering (DLS), and as a result, the Z-average particle size was found to be 8.7 nm (polydispersity index: 0.14). The particle size of the SCNP before and after encapsulation of DACHPt is shown in Fig. 3. The particle size of the SCNP hardly varied before and after encapsulation of DACHPt. The results are summarized in the following table.

### [Measurement apparatus and conditions]

(1) ICP-AES measurement
   Apparatus: Sequential high-frequency plasma light-emitting apparatus ICPE-9000/Shimadzu Corporation
   Pretreatment apparatus: Microwave sample pretreatment apparatus ETHOS EASY/Milestone General K.K.
   Measurement wavelength: 342 nm
   Standard solution: Gadolinium standard solution (Gd 1000) for ICP analysis/FUJIFILM Wako Pure Chemical Corporation
(2) DLS measurement
   Apparatus: Zetasizer NanoZS/Malvern Instruments Ltd.
   Measurement temperature: 25°C
   Sample concentration: 10 mg/mL
   Result: Fig. 3

**[Table 4]**

| Example | Yield (mL) | Pt content (µg/mL) | Pt binding amount per polymer (mol/mol) | Z-average particle size (nm) | Polydispersity index |
|---|---|---|---|---|---|
| 70 | 5 | 720 | 3.4 | 8.7 | 0.14 |

### [Comparative Example 1]

### Preparation of oxaliplatin solution

To 5.58 mL of a 5.9 (w/v)% glucose solution was added 1 mL of 50 mg of ELPLAT I.V. infusion solution (Yakult Honsha Co., Ltd.) to prepare a 5 (w/v)% glucose solution containing 760 µg of oxaliplatin.

### [Test example] Drug efficacy test

A tumor-bearing model obtained by subcutaneously transplanting a mouse colon cancer cell line C26 (American Type Culture Collection) into a female nude mouse (BALB/c-nu nu/nu, 7 weeks old; Charles River Laboratories Japan, Inc.) was used for a drug efficacy test.

A mouse colon cancer cell line C26 subcultured in a CO₂ incubator was suspended in a liquid medium (Dulbecco's Modified Eagle's Medium-high glucose, Sigma-Aldrich Co. LLC), and injected subcutaneously in the back of nude mice so that the number of cells per mouse was 1 × 10⁶/100 µL. Thereafter, the nude mice were raised for about 1 week, and administration of a drug was started when the average value of the tumor volume was grown to about 30 mm³. A DACHPt-encapsulating SCNP (DACHPt-encapsulating SCNP prepared using the copolymer of Example 70) was administered into the tail vein (3 times every other day), and the antitumor effect was evaluated from the tumor volume (4 to 5 mice in 1 group). As a comparison, an oxaliplatin solution (Comparative Example 1) was used, and the oxaliplatin solution was administered in the same manner. The dose of each preparation was 8 mg/kg (3.9 mg/kg in terms of Pt) as the maximum administrable dose for the oxaliplatin solution, and 3 mg/kg in terms of Pt for the DACHPt-encapsulating SCNP.

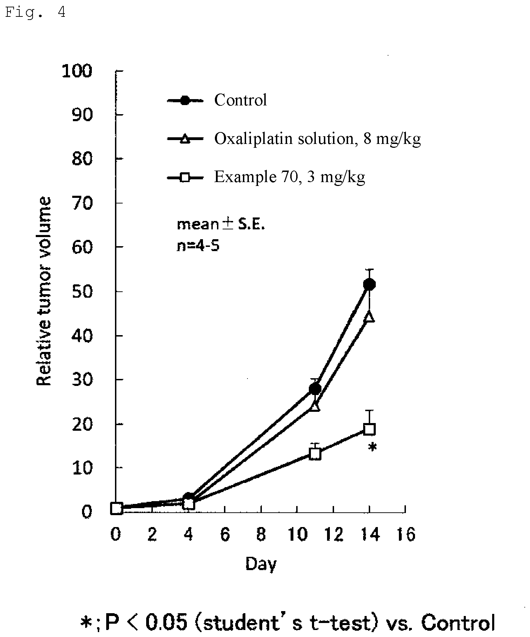

A change in the tumor volume over time is shown in Fig. 4. For the DACHPt-encapsulating SCNP, T/C was 0.4 after 14 days from administration [T/C: ratio of the tumor volume of the drug administration group (T) to that of the control group (C)] . For the oxaliplatin solution (Comparative Example 1), T/C was 1.1 after 14 days from administration. In addition, after 14 days from administration, it was confirmed that the DACHPt-encapsulating SCNP significantly suppressed growth of a tumor as compared with the control (student's t-test). The above results indicate that the DACHPt-encapsulating SCNP has an excellent antitumor effect as compared with the oxaliplatin solution.

## Claims

1. A copolymer comprising structural units represented by the following formulas (A), (B), and (C): 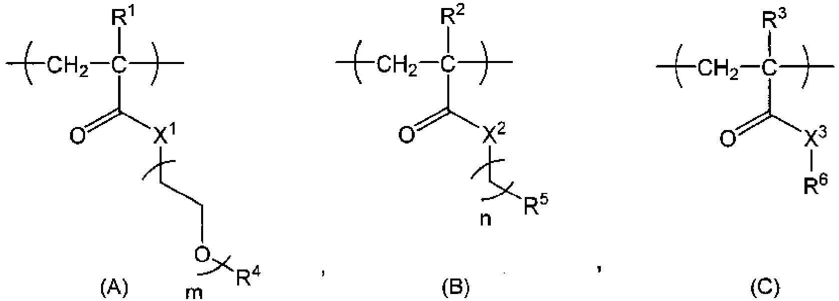 wherein R¹, R², and R³ are the same or different and represent a hydrogen atom or a C₁₋₃ alkyl group, R⁴ represents a C₁₋₃ alkyl group, R⁵ represents a hydrogen atom, a C₁₋₁₈ alkyl group, a 3- to 8-membered cycloalkyl group optionally having a substituent, an adamantyl group, a C₆₋₁₈ aryl group optionally having a substituent, or a 5- to 10-membered heteroaryl group optionally having a substituent, X¹, X², and X³ are the same or different and represent an oxygen atom, a sulfur atom, or N-R⁷, R⁶ represents a hydrogen atom, a leaving group, or a linker, R⁷ represents a hydrogen atom or a C₁₋₃ alkyl group, m represents an integer of 1 to 100, and n represents an integer of 0 to 3.

2. A copolymer formed by polymerization of three monomers represented by the following general formulas (1) to (3): 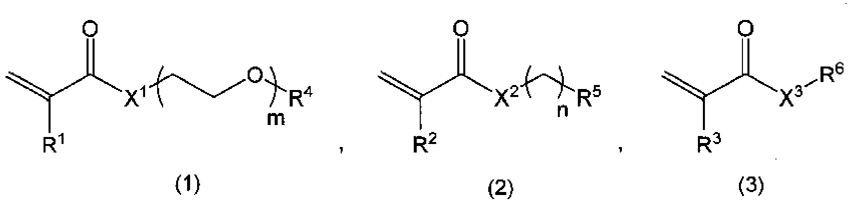 wherein R¹, R², and R³ are the same or different and represent a hydrogen atom or a C₁₋₃ alkyl group, R⁴ represents a C₁₋₃ alkyl group, R⁵ represents a hydrogen atom, a C₁₋₁₈ alkyl group, a 3- to 8-membered cycloalkyl group optionally having a substituent, an adamantyl group, a C₆₋₁₈ aryl group optionally having a substituent, or a 5- to 10-membered heteroaryl group optionally having a substituent, X¹, X², and X³ are the same or different and represent an oxygen atom, a sulfur atom, or N-R⁷, R⁶ represents a hydrogen atom, a leaving group, or a linker, R⁷ represents a hydrogen atom or a C₁₋₃ alkyl group, m represents an integer of 1 to 100, and n represents an integer of 0 to 3.

3. The copolymer according to claim 1 or 2, wherein R¹ is a hydrogen atom.

4. The copolymer according to any one of claims 1 to 3, wherein R² is a hydrogen atom.

5. The copolymer according to any one of claims 1 to 4, wherein R³ is a hydrogen atom.

6. The copolymer according to any one of claims 1 to 5, wherein R⁴ is a methyl group.

7. The copolymer according to any one of claims 1 to 6, wherein R⁵ is a C₆₋₁₈ aryl group optionally having a substituent.

8. The copolymer according to any one of claims 1 to 7, wherein R⁵ is a phenyl group.

9. The copolymer according to any one of claims 1 to 8, wherein R⁶ is a hydrogen atom.

10. The copolymer according to any one of claims 1 to 8, wherein the leaving group of R⁶ is the following formula (4) :

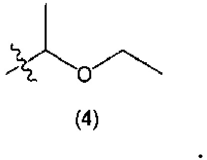

11. The copolymer according to any one of claims 1 to 8, wherein the linker of R⁶ is the following formulas (5) to (7) :

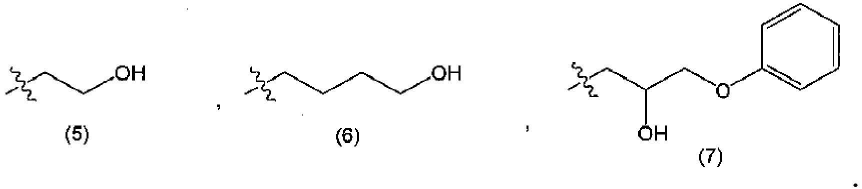

12. The copolymer according to any one of claims 1 to 11, wherein X¹ is an oxygen atom.

13. The copolymer according to any one of claims 1 to 12, wherein X² is an oxygen atom.

14. The copolymer according to any one of claims 1 to 13, wherein X³ is an oxygen atom.

15. The copolymer according to any one of claims 1 to 14, wherein m is an integer of 4 to 22.

16. The copolymer according to any one of claims 1 to 15, wherein n is 1.

17. The copolymer according to any one of claims 1 to 16, wherein a ratio of structural units (A), (B), and (C) is composed of 0.01 to 100 parts by mass of (B) and 0.1 to 100 parts by mass of (C) with respect to 1 part by mass of (A).

18. The copolymer according to any one of claims 2 to 16, wherein 0.01 to 100 parts by mass of monomer (2) and 0.1 to 100 parts by mass of monomer (3) are polymerized with respect to 1 part by mass of monomer (1).

19. The copolymer according to any one of claims 1 to 18, wherein a number average molecular weight is 5 000 to 150 000.

20. A single chain nanoparticle comprising the copolymer according to any one of claims 1 to 19.

21. A pharmaceutical composition comprising the copolymer according to any one of claims 1 to 19.
